# EUROPÄISCHE PATENTSCHRIFT

(11) **EP 0 876 828 B1**
(45) Veröffentlichungstag und Bekanntmachung des Hinweises auf die Patenterteilung: **03.03.2004**
(21) Anmeldenummer: 98250153.8
(22) Anmeldetag: 06.05.1998
(51) Int. Cl.: A61N 1/368

(54) **Vorrichtung zur Erkennung tachykarder Herzrhythmusstörungen**
Device for recognition of tachycardial arrhythmias
Dispositif pour la reconnaissance des arythmies tachycardiques

(30) Priorität: 07.05.1997 US 852738
(43) Veröffentlichungstag der Anmeldung: 11.11.1998
(73) Patentinhaber: BIOTRONIK Mess- und Therapiegeräte GmbH & Co Ingenieurbüro Berlin, 12359 Berlin (DE)
(72) Erfinder: Thong, Tran, Lake Oswego, Oregon 97035 (US); Revishvili, Amiran, Sh.,Prof. Dr., 117588 Moskau 15 (RU)
(74) Vertreter: Eisenführ, Speiser & Partner

(56) Entgegenhaltungen:
- EP-A- 0 597 459
- EP-A- 0 748 638
- US-A- 5 243 980

## Beschreibung

Die Erfindung betrifft eine Vorrichtung gemäß dem Oberbegriff des Anspruchs 1.

Eine solche Vorrichtung ist als eigenständiges Diagnosegerät oder als Bestandteil eines Herzschrittmachers mit Antitachykardie-Betriebsart, Defibrillators, Kardioverters oder eines Kombinationsgerätes mit der Funktion eines der vorgenannten Geräte realisierbar.

Die Therapie tachykarder Herzrhythmusstörungen ist eines der wichtigsten Aufgabengebiete der Kardiologie und speziell ein Hauptanwendungsfeld der Elektrostimulation des Herzens.

Angesichts der Vielfalt der Erscheinungsformen und Ursachen derartiger Störungen sind in den letzten Jahren umfangreiche Anstrengungen unternommen worden, die Voraussetzungen für einen therapeutischen Erfolg durch feinere Klassifizierung der tachykarden Arrhythmien und exaktere Zuordnung zu typischen Defekten des kardialen Reizleitungssystems zu verbessern. Aufgrund der vergleichsweise weiten Verbreitung und der Behandlungsbedürftigkeit einerseits und der guten Chancen für ihre erfolgreiche Behandlung mittels spezieller Schrittmacherimpulsfolgen andererseits hat sich dabei die sichere Unterscheidung pathologischer ventrikulärer Tachykardien von anderen Arrhythmieformen - etwa von physiologischen Tachykardien, ventrikulären Fibrillationen oder supraventrikulärer Tachykardie - , auch unter differenzierten Umständen, als besonders wichtiges Problem herauskristallisiert.

In EP 0 094 758 A2 wurde vorgeschlagen, aus dem Vergleich eines aktuellen Zeitintervalls zwischen aufeinanderfolgenden Herzschlägen mit einem vorher bestimmten Mittelwert einerseits und mit einem vorgegebenen (Fest-)Wert andererseits ein Kriterium für das Vorliegen einer pathologischen Tachykardie zu gewinnen. Damit wurde gegenüber den frühesten Nachweisverfahren, die sich ausschließlich auf die Auswertung der aktuellen Herzrate stützten und dabei physiologische nicht von pathologischen Tachyarrhythmien unterscheiden konnten, zwar ein Fortschritt erzielt. Für eine exakte Klassifizierung der verschiedenen Tachykardietypen reicht die Aussagekraft dieses Verfahrens jedoch nicht aus.

In Weiterentwicklung dieses Verfahrena wird in DE 44 39 256 A1 vorgeschlagen, die relative Verteilung von als Fibrillations-Intervalle bzw. von als Tachykardieintervalle anzusehenden Herzschlagintervallen innerhalb vorbestimmter Zeitbereiche zur Klassifizierung ds vorherrschenden Arrhythmietyps zu nutzen. Damit soll der Tatsache Rechnung getragen werden, daß die Intervallängen sich bei ventrikulärer Tachykardie einerseits und Fibrillationen andererseits in der Praxis häufig erheblich überlappen. Da letztlich jedoch weiterhin allein aufgrund der Intervalle zwischen Kammeraktionen (der "RR-Intervalle") klassifiziert wird, kann dieses Vorgehen das Problem nicht an der Wurzel packen.

Später ist verschiedentlich - wie etwa in EP 0 302 577 A2 - die Signalform des (auf konventionelle Weise oder intrakardial) aufgenommenen EKG als Unterscheidungs- und sogar Vorhersagekriterium vorgeschlagen worden. Die EKG-Signalform widerspiegelt jedoch das Auftreten einer ventrikulären Tachykardie nicht hinreichend verläßlich, und die zu einer einigermaßen aussagefähigen Auswertung benötigte hohe Signalauflösung erfordert einen sehr hohen Meß- und Verarbeitungsaufwand, der bei einem implantierten Schrittmacher kaum realisierbar ist.

Bei der in EP 0 580 128 A1 vorgeschlagenen Lösung zur Tachykardieklassifizierung wird der Aufwand durch das zusätzliche Vorsehen eines Aktivitätssensors und von Verarbeitungsmitteln für dessen Signale noch weiter erhöht.

Eien ganze Reihe anderer Lösungsansätze - die hier nicht im einzelnen erörtert werden sollen - bedient sich der Analyse der räumlichen Ausbreitung bzw. Korrelation von Depolarisationen im Herzgewebe. Dies erfordert die Implantation einer Vielzahl von Elektroden zur Signalerfassung - zum Teil sogar perikardial - und hat schon aus diesem Grunde nur geringe Realisierungschancen.

In US 4 860 749 wird ein Verfahren zur Unterscheidung einer ventrikulären von einer Sinus- bzw. anderen supraventrikulären Tachykardie beschrieben, bei dem die atriale und die ventrikuläre Herzrate (deren jeweilige Kehrwerte werden im weiteren auch als "PP-Intervall" bzw. "RR-Intervall" bezeichnet) sowie das AV-Intervall (im weiteren teilweise auch als "PR-Intervall" bezeichnet) gemessen werden. Liegt das RR-Intervall innerhalb eines vorbestimmten Bereiches und ist es kürzer als das PP-Intervall, wird der Zustand ohne weiteres als ventrikuläre Tachykardie klassifiziert. Sind die atriale und die ventrikuläre Rate infolge 1:1-AV-Überleitung oder retrograder Überleitung etwa gleich, wird das gemessene AV-Intervall einem Vergleich mit einem vorgegebenen Wert ("Sinus-AV-Intervall") unterzogen und aus dem Ergebnis des Vergleichs das Klassifizierungskriterium gewonnen.

Dieses Vorgehens bedient sich auch die in US 5 107 850 vorgeschlagene Lösung, bei der sich jedoch eine weitere Verarbeitung anschließt: Für den Fall, daß das gemessene AV-Intervall länger als das vorbestimmte ist, wird die "Regularität" (ein Maß für die zeitliche Konstanz) der gemessenen AV-Intervalle sowie die ventrikuläre Rate (deren Kehrwert wird nachfolgend auch als RR-Intervall bezeichnet) ermittelt und ausgewertet, wofür noch ein zweiter vorgegebener Wert des AV-Intervalls benötigt wird. Aufgrund zusätzlicher Messung der atrialen Rate und der Bestimmung der Regularität auch der atrialen und ventrikulären Rate kann auch eine atriale Tachyarrhythmie klassifiziert werden.

Auf sehr ähnliche Weise wird auch bei US 5 383 910 vorgegangen.

Bei den zuletzt genannten Verfahren bringt die Vorgabe eines oder sogar zweier verschiedener AV-Werte, die üblicherweise bei der Erstprogrammierung der Vorrichtung erfolgt, zusätzliche Anpassungsparameter ins Spiel, die die Effizienz des angewandten Algorithmus erheblich einschränken können, wenn sie von vornherein unglücklich gewählt oder - etwa im Ergebnis physiologischer Veränderungen beim Patienten - unbemerkt ungültig werden.

In US 5 325 856 wird ein Verfahren zur Unterscheidung zwischen ventrikulären und supraventrikulären Tachykardien vorgeschlagen, das auf einem Vergleich der zeitlichen Divergenz der PR- und der RR-Werte mit zwei vorbestimmten Schwellwerten beim Einsetzen der Tachykardie beruht. Auch hierbei spielen weitgehend willkürlich gewählte Parameter eine entscheidende Rolle für die Klassifizierung der Arrhythmiezustände, was die Gefahr von Fehlzuordnungen in sich birgt.

US 5 327 900 beschreibt ein Verfahren zur Unterscheidung zwischen pathologischen und physiologischen Tachykardien bei vergleichbarer atrialer und ventrikulärer Rate, das auf der Zuordnung des gemessenen AV-Intervalls zu einem vorgegebenen AV-Zeitfenster beruht, welches anhand des AV-Intervalls bei normalem Sinusrhythmus bestimmt worden ist. Dieser Algorithmus ist vergleichsweise einfach; es liegen jedoch bislang keine Belege für eine hinreichende Unterscheidungs-Effizienz vor.

Ein weitaus aufwendigeres Verfahren, bei dem zur Ermittlung der Therapierbarkeit einer festgestellten Tachyarrhythmie im Falle annähernder übereinstimmung zwischen atrialer und ventrikulärer Rate eine ganze Reihe von zusätzlichen Kriterien (zum RR-Intervall und zum Verhältnis zwischen PP- und RR-Intervall) angewandt wird, wird in US 5 379 776 beschrieben.

Aus J.M.JENKINS et al., "A Single Atrial Extrastimulus Can Distinguish Sinus Tachycardia from 1:1 Paroxysmal Tachycardia", PACE Vol. 9/II (1986), sowie J.M. JENKINS, S. CAS-WELL, "Detection Algorithms in Implantable Cardioverter Defibrillators", Proc. IEEE 84, No. 3, 428 (1996), ist ein Verfahren bekannt, bei dem durch frühe atriale Stimulation und eine Beobachtung der ventrikulären Reaktion zwischen Sinus-Tachykardie einerseits und anderen tachykardie-Arten, etwa ventrikulärer Tachykardie mit retrograder 1:1-Überleitung, unterschieden werden kann. Die mit diesem Verfahren mögliche Unterscheidung ist jedoch von geringem klinischem Wert; insbesondere ist für den Betrieb eines Kardioverters/Defibrillators eine Unterscheidung zwischen behandlungsbedürftiger ventrikulärer Tachykardie (VT) und nicht behandlungsbedürftiger supraventrikulärer Tachykardie (SVT) nötig.

In EP 0 597 459 A2 wird ein hierauf aufbauendes Verfahren beschrieben, bei dem im Falle einer Übereinstimmung von RRund PP-Intervall zunächst ein Vergleich der Länge des AV- bzw. PR-Intervalls mit einem vorgegebenen Basiswert und schließlich - falls dieser nicht zu einem schlüssigen Ergebnis führt - eine Test-Stimulationimpulsfolge mit vorbestimmter, erhöhter Impulsrate abgegeben und die Klassifizierung anhand der stimulierten Herzantwort, insbesondere zeitlicher Änderungen der RR-Intervalle sowie der Größe des AV-Intervalls, vorgenommen wird. Dieses Vorgehen birgt die Gefahr der Induzierung oder Beschleunigung einer ventrikulären Tachykardie in sich.

In EP 0 647 150 A1 ist eine Vorrichtung zur Unterscheidung zwischen ventrikulären und supraventrikulären Tachykardien beschrieben, bei der in Synchronisation mit der R-Welle die mit dem Sinusknoten und/oder AV-Knoten zusammenhängenden Fettpolster stimuliert werden und die Klassifikation der Tachykardie in Abhängigkeit von der ermittelten Änderung des Herzrhythmus erfolgt. Dies erfordert die Implantation zusätzlicher Elektroden neben einer herkömmlichen Stimulations-Elektrodenleitung und bringt daher eine Erhöhung des Operationsaufwandes, der Kosten und der Risiken für den Patienten mit sich.

Der Erfindung liegt daher die Aufgabe zugrunde, eine einfach aufgebaute, leicht zu implantierende und minimale Risiken für den Patienten bedingende Vorrichtung zur Erkennung verschiedener tachykarder Herzrhythmusstörungen anzugeben, mit der speziell eine Unterscheidung zwischen zwischen ventrikulären und supraventrikulären Tachykardien erreicht werden kann.

Die Aufgabe wird durch eine Vorrichtung mit den Merkmalen des Anspruchs 1 gelöst.

Die Erfindung schließt den Gedanken ein, eine Stimulation zur Induzierung einer zur eindeutigen Tachykardie-Klassifizierung brauchbaren Herzantwort unter Verzicht auf zusätzliche Elektroden und Hochrate-Impulsfolgen durchzuführen. Sie schließt weiter den Gedanken ein, eine Vorrichtung mit Mitteln anzugeben, um zunächst aufgrund der mit je einem Fühler im Atrium und im Ventrikel aufgenommenen Signale spontaner Herzaktivität eine erste Klassifizierung auszuführen, bei der die Länge der RR- und der PP-Intervalle sowohl betragsmäßig als auch in ihrem Verhältnis zueinander ausgewertet wird. Ergibt sich hierbei eine so-genannte 1:1-Tachykardie (bei der die RR-Intervalle annähernd gleich den PP-Intervallen sind), wird unter weitestgehender Nutzung der vorhandenen Mittel eine weitergehende Klassifizierung aufgrund des Ansprechens des Vorhofes auf eine Folge ventrikulärer Einzel-Stimuli mit sukzessive gegenüber dem Zeitpunkt der spontanen Kammeraktionen verschobenem Ausgabezeitpunkt (VES = "ventricular early stimulus") vorgenommen. Folgen die erfaßten Vorhofaktionen der zeitlichen Verschiebung der Stimuli gegenüber den spontanen Kammeraktionen, so liegt eine ventrikuläre Tachykardie (VT) mit retrograder Überleitung vor. Bleiben die PP-Intervalle hingegen im wesentlichen konstant, so liegt eine supraventrikuläre Tachykardie (SVT) vor, und ist keine eindeutige Tendenz auszumachen, zeigt dies das Vorliegen gelegentlicher atrialer Überleitung und spricht ebenfalls für eine SVT.

Zwischen den Einzel-Stimuli der Folge wird jeweils eine Anzahl spontaner Kammeraktionen abgewartet, um eine Erholung ("recovery") der spontanen Herzaktivität sicherzustellen.

Die erfindungsgemäße Vorrichtung ist in Herstellung und Implantation vergleichsweise unaufwendig und bringt bei der Implantation und im Betrieb für den Patienten keine höhere Belastung mit sich als ein herkömmlicher Herzschrittmacher/Kardioverter.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein Funktions-Blockschaltbild einer Vorrichtung nach einer Ausführungsform der Erfindung,
Figur 2 eine schematische Darstellung zum Aufbau der Vergleichereinheit 207 der Vorrichtung nach Fig. 1,
Figur 3 eine Detaildarstellung zum Aufbau der Stimulations-Steuereinrichtung 3 der Vorrichtung nach Fig. 1,
Figur 4 eine Detaildarstellung zum Aufbau der Vorhofintervall-Auswertungseinheit 208b nach Fig. 1,
Figuren 5a und 5b ein Flußdiagramm des Ablaufes bei Unterscheidung einer ventrikulären von einer supraventrikulären Tachykardie mittels einer Vorrichtung nach Fig. 1 und
Fig. 6 eine grafische Darstellung zum Funktionsprinzip.

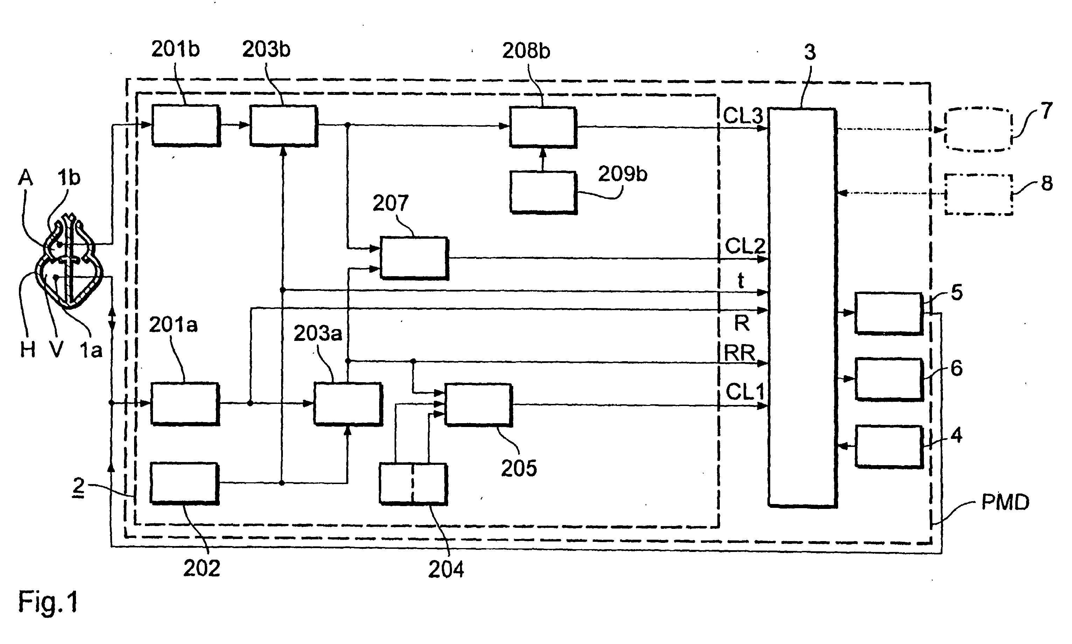

In Figur 1 ist schematisch der Aufbau einer über eine Kammerelektrode 1a und eine Vorhofelektrode 1b als endokardiale Signalaufnehmer mit einem Herzen H verbundenen Vorrichtung zur Tachykardieklassifizierung, speziell zur Unterscheidung zwischen ventrikulärer und supraventrikulärer Tachykardie, nach einer Ausführungsform der Erfindung anhand der wesentlichen Funktionselemente dargestellt.

Die Vorrichtung umfaßt eine Erfassungs- und Auswertungseinheit 2 und eine Steuereinheit (einen Controller) 3 und wird entsprechend einem weiter unten erläuterten, in einem Programmspeicher 4 gespeicherten Ablauf gesteuert. Aufgrund von durch die Vorrichtung ausgegebenen Klassifizierungssignalen wird wahlweise ein als solcher bekannter Schrittmacher-Impulsgenerator 5 oder Defibrillator 6 zu einer geeigneten Elektrotherapie angesteuert. Die Baugruppen 2 bis 6 bilden (mit an sich bekannten und daher hier nicht gezeigten weiteren Komponenten, wie einer Stromversorgung) einen kombinierten Schrittmacher/Defibrillator PMD.

Die von der Erfassungs- und Auswertungseinheit 2 ausgegebenen Klassifizierungsdaten können durch eine optional vorgesehene (mit einer gestrichelten Linie symbolisierte) Anzeigeeinheit 7 dargestellt und somit erforderlichenfalls auch unabhängig von der Einleitung einer Elektrotherapie - beispielsweise zur Festlegung einer medikamentösen Therapie - genutzt werden. Ebenfalls optional ist eine Eingabeeinheit 8 zur Eingabe von die Ausführung einer konkreten Klassifizierungsprozedur betreffenden, patientenspezifischen Daten vorgesehen.

Zumindest Teile der Erfassungs- und Auswertungseinheit 2 und/oder der Controller 3 können für spezielle Anwendungen - etwa für den begleitenden Einsatz bei herzchirurgischen Eingriffen auch in einem separaten Gerät - unabhängig von einem Herzschrittmacher und/oder Kardioverter - realisiert sein.

In beiden Fällen wird die praktische Ausführung in vorteilhafter Weise in einer Mikroprozessorstruktur oder auch partiell auf ASIC(Kundenwunschschaltungs)-Basis erfolgen, wobei die nachfolgend erläuterten Funktionselemente zumindest teilweise durch Software realisiert sind.

Wie in Figur 1 dargestellt, weist die Erfassungs- und Auswertungseinheit 2 einen mit der atrialen Abfühlelektrode 1b verbundenen Abfühlverstärker 201b zum Abfühlen elektrischer Aktivität (der P-Wellen) im Vorhof A des Herzens H und einen mit der ventrikulären Abfühlelektrode 1a verbundenen Abfühlverstärker 201a zum Abfühlen elektrischer Aktivität (insbesondere der R-Wellen bzw. QRS-Komplexe) in der Herzkammer V des Herzens H auf. Weiter ist ein Zeit- bzw. Taktgeber 202 zur Erfassung des Zeitpunktes des Auftretens der jeweiligen elektrischen Aktivität vorgesehen.

Eine eingangsseitig mit der Kammerelektrode 1a sowie dem Zeitgeber 202 verbundene Kammerintervall-Erfassungseinheit 203a - die in der einfachsten Ausführung im Grunde einen R-Wellen-getriggerten Zähler für die Impulse des Zeitgebers darstellt, in der bevorzugten Ausführung jedoch eine Mittelwertbildung der Zählwerte über eine Anzahl von RWellen ausführt - ermittelt die (gemittelten) Zeitintervalle zwischen jeweils aufeinanderfolgenden elektrischen Aktivitäten in der Herzkammer (RR-Intervalle). Analog dient eine eingangsseitig mit der Atrium-Elektrode 1b sowie dem Zeitgeber 202 verbundene Vorhofintervall-Erfassungseinheit (in der einfachsten Ausführung ein P-Wellen-getriggerter Zähler) 203b zur Ermittlung der - ggfs. gemittelten - Zeitintervalle zwischen jeweils aufeinanderfolgenden elektrischen Aktivitäten im Vorhof (PP-Intervalle). Eine Mittelung der gemessenen Zeitintervalle in der ersten und zweiten Berechnungseinheit 203a, 203b erfolgt auf an sich bekannte Weise durch eine (nicht dargestellte) Integrations- bzw. Mittelungseinrichtung, wobei beispielsweise eine Anzahl von vier erfaßten Herzaktionen zugrundegelegt wird.

Ein Zwei-Bereichs-Schwellwertspeicher 204 dient zur Speicherung eines unteren und eines oberen Schwellwertes für die Kammerintervalle, von denen der obere einen Bereich normaler Herzaktivität von einem Bereich tachykarder Rhythmusstörungen abgrenzt und der untere einen Bereich als ventrikuläre Fibrillation zu klassifizierender Herzaktivität nach oben hin abgrenzt. Mit den Ausgängen der Kammerintervall-Erfassungseinheit 203a und des Schwellwertspeichers 204 ist ein Zwei-Stufen-Komparator 205 zum Vergleich der Kammerintervalle (bzw. Intervall-Mittelwerte) mit dem unteren und oberen Schwellwert und zur Ausgabe eines das Vergleichsergebnis - d.h. die Lage der ermittelten RR-Intervalle in einem der oben erwähnten Bereiche - kennzeichnenden ersten Klassifizierungssignals CL1 verbunden.

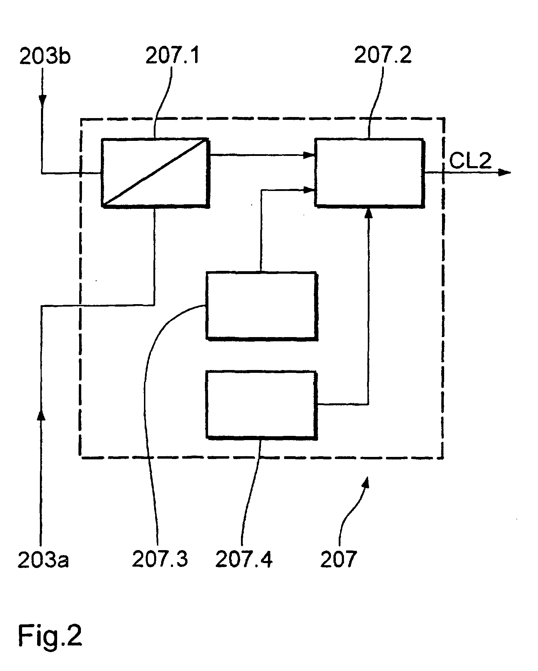

Mit dem Ausgang der Vorhofintervall-Erfassungseinheit 203b ist eine weitere Komparatorstufe 207 zum Vergleich der Atrium-Zeitintervalle mit den Ventrikel-Zeitintervallen und zur Ausgabe eines das Vergleichsergebnis kennzeichnenden zweiten Klassifizierungssignals CL2 verbunden. Der Aufbau der Komparatorstufe 207 ist in Fig. 2 genauer gezeigt und wird weiter unten beschrieben.

Weiterhin ist mit dem Ausgang des Vorhofintervall-Erfassungseinheit 203b eine Vorhofintervall-Auswertungseinheit 208b verbunden, die zur Ausgabe eines das Ergebnis einer weiter unten genauer beschriebenen Auswertung kennzeichnenden dritten Klassifizierungssignals CL3 ausgebildet ist. Ein Auswertungsvorgang wird jeweils über ein Steuersignal vom Controller 3 ausgelöst. Ein Kriterienspeicher 209b dient zur Speicherung eines vorgegebenen Auswertungskriteriums.

Neben den Klassifizierungssignalen CL1 bis CL3 werden dem Controller 3 ein die Erfassung einer Kammeraktion kennzeichnendes Ausgangssignal R der Kammeraktions-Erfassungseinrichtung 201a, das Zeit- bzw. Taktsignal t des Zeitgebers 202 und das Ausgangssignal RR der Kammerintervall-Erfassungseinrichtung 203a direkt zugeführt. Die Verarbeitung dieser Signale im Controller 3 und die Ablaufsteuerung werden ebenfalls weiter unten beschrieben.

Fig. 2 zeigt - wiederum in einer schematischen Darstellung - den Aufbau der Komparatorstufe 207.

Mit den Ausgängen der (in dieser Figur nicht gezeigten) In-. tervall-Erfassungseinheiten 203a und 203b sind die beiden Eingänge einer Dividierstufe 207.1 verbunden, die eine Division des jeweils zugeführten RR-Intervalles durch das zugehörige PP-Intervall ausführt und deren Ausgang mit einem Eingang einer Vergleicherstufe 207.1 verbunden ist, dem sie den errechneten aktuellen Wert dieses Quotienten zuführt. Der andere Vergleichssignaleingang der Vergleicherstufe ist mit einem Quotientenspeicher 207.3 verbunden, in dem fest vorgegebene ganzzahlige Werte (insbesondere der Wert 1) für den RR/PP-Quotienten gespeichert sind und der daher als Festwertspeicher (ROM) ausgeführt sein kann. In einem weiteren, bevorzugt als reprogrammierbarer Speicher (etwa EE-PROM) ausgebildeten, Speicher 207.4 ist ein Quotienten-Toleranzwert gespeichert, der über einen dritten Eingang an der Vergleicherstufe 207.2 bereitgestellt wird. Das an deren Ausgang abgegebene zweite Klassifizierungssignal CL2 drückt - in den Grenzen des vorgegebenen Toleranzbereiches - somit aus, ob der Quotient RR-Intervall/PP-Intervall ganzzahlig ist, insbesondere annähernd den Wert 1 hat.

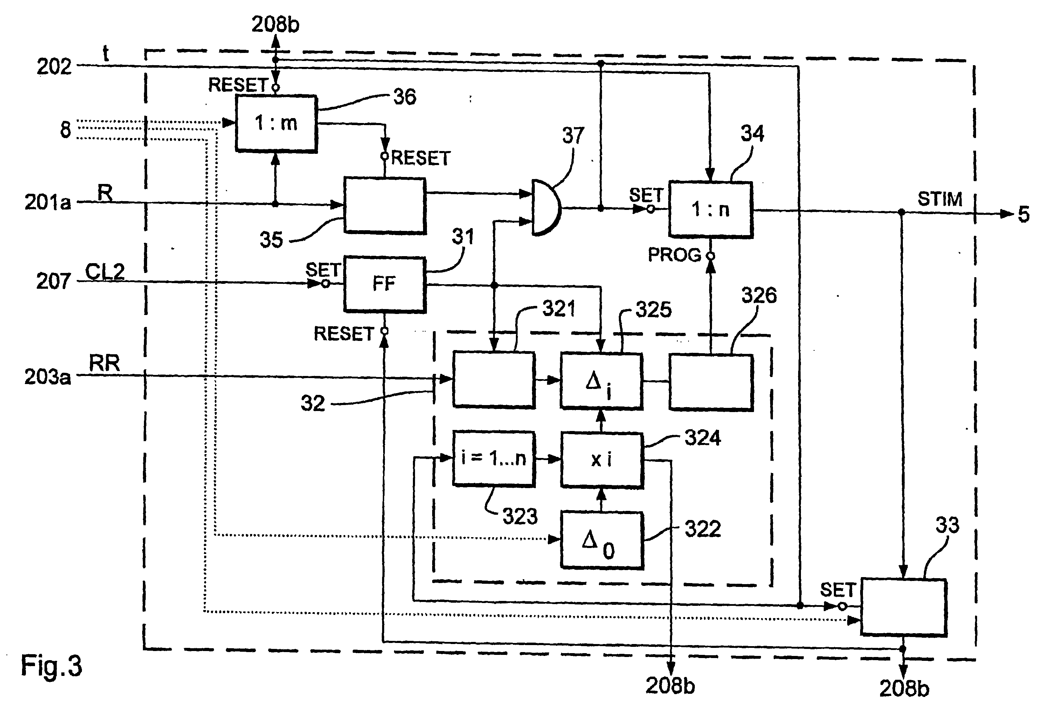

In Fig. 3 sind in Form eines vereinfachten Blockschaltbildes für die Ausführung einer Ausführungsform der Erfindung wesentliche Komponenten des Controllers 3 gezeigt.

Ein über seinen Setzeingang mit dem Ausgang der Komparatorstufe 207 (Fig. 1 und 2) verbundenes Flipflop 31 ist ausgangsseitig mit dem Steuereingang einer Stimulierungszeitpunkt-Berechnungseinheit 32 und über seinen Rücksetzeingang mit dem Ausgang eines programmierbaren 1:n-Teilers 33 verbunden. Der Dateneingang der Stimulierungszeitpunkt-Berechnungseinheit 32 (deren Aufbau weiter unten skizziert wird), ist mit dem Ausgang der Kammerintervall-Erfassungs einrichtung 203a verbunden, und ihr Ausgang ist mit dem Programmiereingang eines programmierbaren Zählers 34 verbunden. Dessen Dateneingang ist mit dem Zeitgeber 202 (Fig. 1) und dessen Ausgang mit dem Schrittmacher-Impulsgenerator 5 verbunden und führt diesem bei Erreichung des programmierten Zählwertes ein Aktivierungssignal zu. Jede Zählung durch den Zähler 34 wird über einen Steuereingang initiiert, der wie folgt angesteuert wird.

Der Controller 3 weist weiter ein eingangsseitig mit dem Ausgang der Kammeraktions-Erfassungseinheit 201a (Fig. 1) verbundenes Sperrglied 35 auf, das durch einen programmierbaren 1:m-Teiler 36 zurückgesetzt wird. Der Ausgang des Sperrgliedes 35 ist mit einem Eingang eines UND-Gatters 37 verbunden, dessen anderer Eingang zum Ausgang des Flipflop 31 geführt ist. Der Ausgang des UND-Gatters 37 ist mit Steuereingängen der Teiler 33 und 36, des Zählers 34 und der Stimulationszeitpunkt-Berechnungseinrichtung 32 verbunden und steuert diese Komponenten an.

Die Berechnungseinrichtung 32 umfaßt einen mit dem Ausgang der Kammerintervall-Erfassungseinrichtung 203a verbundenen Kammerintervallspeicher 321, einen programmierbaren Basisdekrementspeicher 322 und einen eingangsseitig mit dem Ausgang des UND-Gatters 37 verbundenen Zähler 323 sowie eine mit dessen Ausgang sowie dem Dekrementspeicher verbundene Multiplikatorstufe 324 und eine mit deren Ausgang sowie dem Kammerintervallspeicher 321 verbundene Substraktionsstufe 325. Deren Ausgang ist mit der Vorhofintervall-Auswertungseinheit 208b (Fig. 1) sowie einem Stimulationsabstandsspeicher 326 verbunden, dessen Ausgang den Ausgang der Berechnungseinheit 32 bildet.

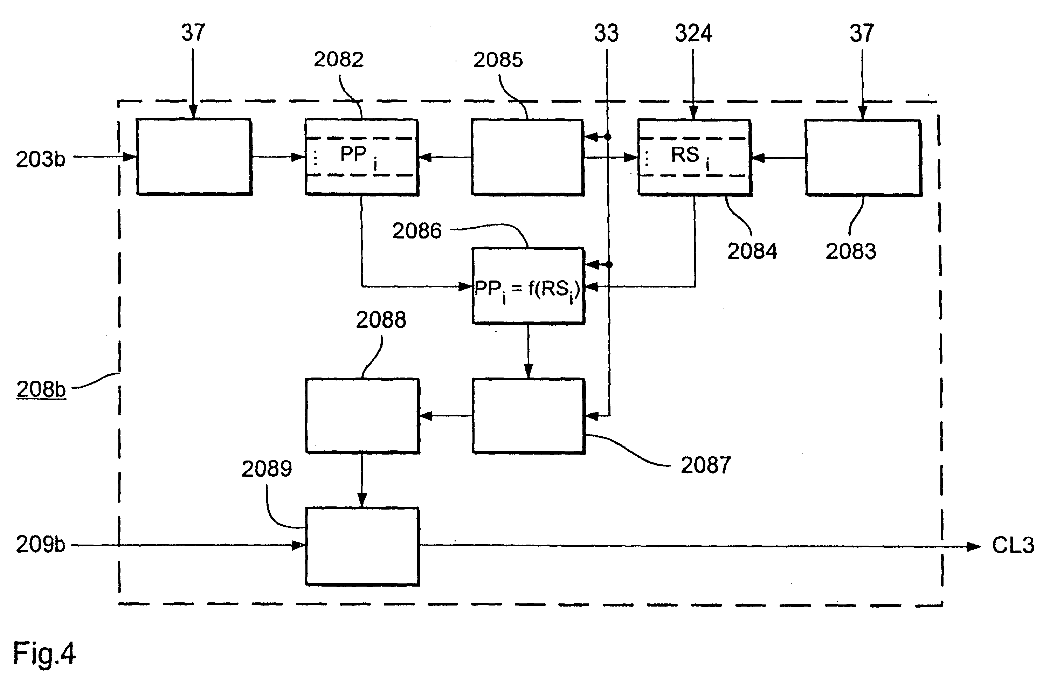

Fig. 4 zeigt - ebenfalls als schematisches Funktions-Blockschaltbild - den Aufbau der Auswertungseinheit 208b aus Fig. 1 in einer Ausführungsform. Sie umfaßt einen über eine Speicherschreibsteuerung 2081 mit dem Ausgang der Vorhofintervall-Erfassungseinheit 203b (Fig. 1) verbundenen Vorhofintervallspeicher 2082 und einen über eine separate Speicherschreibsteuerung 2083 mit dem Ausgang der Multiplikatorstufe 324 (Fig. 3) verbundenen Kammerintervall-Dekrementspeicher 2084. Der Vorhofintervallspeicher 2082 und der Kammerintervall-Dekrementspeicher 2084 sind als serielle Mehrbereichsspeicher zu Speicherung einer vorgegebenen Anzahl n von Abstandsdifferenzwerten PPᵢ vorbestimmter Vorhofaktionen bzw. von hierzu korrespondierenden voreingestellten Dekrementwerten Δᵢ bzw. RSᵢ der Kammeraktions-Stimulus-Intervalle ausgebildet.

Die Speicherschreibsteuerungen 2081, 2083 werden durch das oben erwähnte UND-Gatter 37 angesteuert. Eine durch den Teiler 33 (Fig. 3) angesteuerte Speicherlesesteuerung 2085 bewirkt ein Auslesen der Speicherinhalte in eine Korrelatorstufe 2086 bei Änderung des Ausgangszustandes des Teilers, und auf gleiche Weise werden die Korrelatorstufe 2086 selbst, eine dieser nachgeschaltete Approximationseinheit 2087 und ein dieser nachgeschaltetes Differenzierglied 2088 aktiviert. Der Ausgang des letzteren ist mit einem Eingang eines Anstiegs-Komparators 2089 verbunden, dessen anderer Eingang mit dem Kriterienspeicher 209b (Fig. 1) verbunden ist und dessen Ausgang das Klassifizierungssignal CL3 bildet.

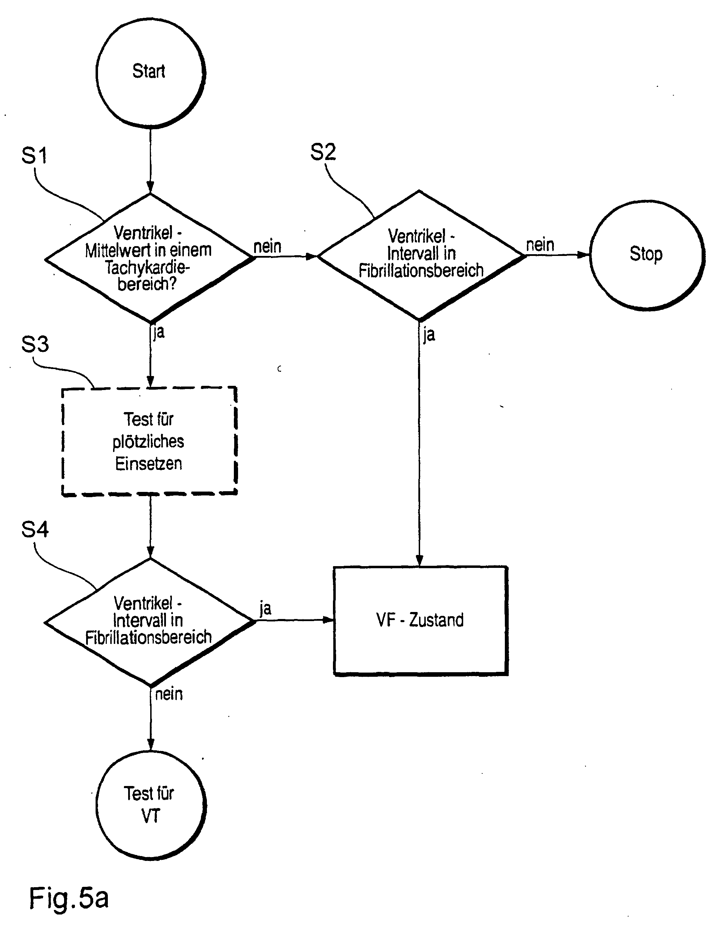

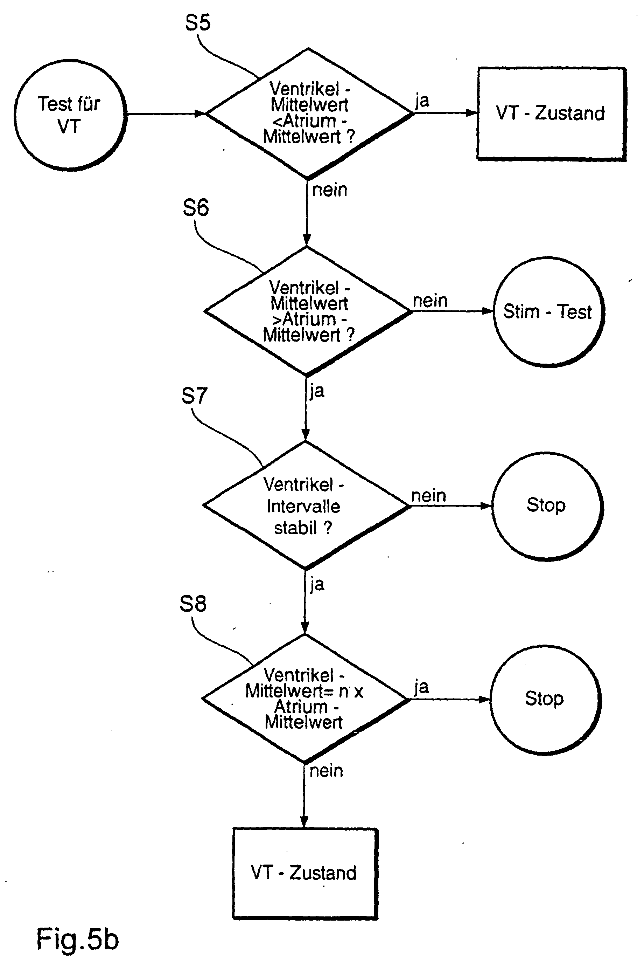

Der Betrieb der oben in ihrem grundsätzlichen Aufbau beschriebenen Vorrichtung wird nachfolgend unter zusätzlicher Bezugnahme auf die Figuren 5a und 5b erläutert, die zusammen ein Flußdiagramm des Vorgehens zum Nachweis einer VT bzw. zu deren Unterscheidung von einer SVT zeigen. Dieses Vorgehen ist besonders vorteilhaft bei gleichzeitigem Vorliegen einer atriellen Tachyarrhythmie, die mit gleicher oder höherer Rate abläuft wie die im Ventrikel nachgewiesene und zu klassifizierende Tachyarrhythmie.

Nach dem Start der Prozedur wird in der ersten Berechnungseinheit 203a der zeitliche Abstand (das RR-Intervall) zweier aufeinanderfolgender, auf an sich bekannte Weise durch die Kammerelektrode 1a abgefühlter und in der zugeordneten Erfassungseinheit 201a aufbereiteter Kammeraktionen (R-Wellen-Signale) bestimmt.

In zwei aufeinanderfolgenden Schritten S1 und S2 wird über den Vergleicher 205 zunächst in einem Vergleich mit den im Speicher 204 gespeicherten Intervall-Schwellwerten eine Zuordnung zu einem der Intervall- bzw. Ratenbereiche
(1) "normaler" Sinusaktivität (relativ lange Intervall-bzw. niedrige Ratenwerte) oder
(2) näher zu klassifizierender Tachyarrhythmiezustände (für einen Tachyarrhathmiezustand mittelgroße Intervall- bzw Ratenwerte) oder
(3) Fibrillations-Bereich (sehr kurze Intervall- bzw. hohe Ratenwerte)
vorgenommen. Die beiden Schwellwerte sind patientenspezifisch und vom Arzt zu programmieren.

Wird festgestellt, daß der Kammerintervallwert weder in einem Tachykardiebereich (2) ("Nein" im Schritt S1) noch im Fibrillationsbereich (3) ("Nein" im Schritt S2), mithin im Bereich (1), liegt, kann von der Einleitung einer Elektrotherapie abgesehen werden ("Stop" nach S2). Liegt er hingegen im Bereich (3) ("Ja" im Schritt S2), wird in der Regel über den Controller 3, der das entsprechende Klassifizierungssignal CL1 empfangen und verarbeitet hat, der Defibrillator 6 angesteuert und eine Defibrillation bzw. Kardioversion eingeleitet. Ggfs. kann der Arzt auch, nachdem ihm als Klassifizierungsergebnis auf der Anzeige 7 das Vorliegen von Herzkammerflimmern angezeigt wurde, eine Medikamentengabe vornehmen.

Liegt der aktuelle RR-Intervall- bzw. ventrikuläre Ratenwert im Bereich (2) oder (3) ("Ja" im Schritt S1), so kann in einem (optionalen und daher gestrichelt dargestellten) Schritt S3 zusätzlich geprüft werden, ob die Tachyarrhythmie plötzlich eingesetzt hat. (Die hierzu benötigten Komponenten sind in Fig. 1 nicht dargestellt)

Schritt S4 in Fig. 2a steht dafür, daß nach der Prüfung auf plötzliches Einsetzen nochmals geprüft wird, ob der RR-Intervallwert im o.g. Bereich (3) liegt. Trifft dies zu ("Ja" im Schritt S4), liegt eine ventrikuläre Fibrillation "VF-Zustand" vor, die so zu therapieren ist wie oben (für "Nein" im Schritt S2) erwähnt. Anderenfalls ("Nein" im Schritt S4) werden zur Klassifizierung der Tachyarrhythmie weitere Kriterien geprüft ("Test für VT"); siehe weiter in Fig. 5b.

In einem Schritt S5 wird in der Komparatorstufe 207 ein Vergleich der RR-Intervalle (Mittelwerte) mit den am Ausgang der Vorhofintervall-Erfassungseinheit 203b bereitstehenden PP-Intervallen (Mittelwerten) ausgeführt. Sind die RR-Intervalle kürzer als die PP-Intervalle ("Ja" im Schritt S5), liegt eine ventrikuläre Tachykardie ("VT-Zustand") vor.

Sind die RR-Intervall-Mittelwerte nicht kürzer als die PPIntervall-Mittelwerte ("Nein" im Schritt S5), wird im Flußdiagramm zu einem Schritt S6 weitergegangen, in dem geprüft wird, ob sie länger als die PP-Intervall-Mittelwerte sind. (In der Praxis kann dieser Schritt in einem Arbeitsgang mit S5 ausgeführt werden.) Ist dies nicht der Fall ("Nein" im Schritt S6), wird mit einem "Stim-Test" fortgefahren. Trifft es hingegen zu ("Ja" im Schritt S6), wird zum Schritt S7 weitergegangen.

Im Schritt S7 wird geprüft, ob die RR-Intervalle zeitlich stabil sind. Hierzu werden bevorzugt die Differenzen zwischen dem aktuellen und jedem der drei vorhergehenden RR-Intervall-Mittelwerte mit einem vorgegebenen Stabilitätskriterium verglichen, und Stabilität wird als gegeben angenommen, wenn in allen drei Vergleichen das Kriterium erfüllt ist.) Sind die RR- bzw. Ventrikel-Intervalle nicht stabil ("Nein" im Schritt S7), wird der Test mit dem Ergebnis abgebrochen ("Stop"), daß keine ventrikuläre Tachykardie vorliegt. Sind sie hingegen stabil ("Ja" im Schritt S7), wird zu einem Schritt S8 weitergegangen. Die für diesen Schritt benötigten Komponenten sind in Fig. 1 wiederum nicht gezeigt.

Im Schritt S8 wird in der Komparatorstufe 207 geprüft, ob der vom Ausgang der ersten Berechnungseinheit 203a abgegriffene RR-Intervall-Mittelwert ein ganzzahliges Vielfaches des vom Ausgang der zweiten Berechnungseinheit 203b abgegriffenen PP-Intervall-Mittelwertes ist. Dieser Vorgang ist oben unter Bezugnahme auf Fig. 2 beschrieben. Wird festgestellt, daß der RR-Mittelwert ein ganzzahliges Vielfaches des PP-Mittelwertes ist - insbesondere gleich diesem - ist ("Ja" im Schritt S8), so wird der Test abgebrochen ("Stop") - wiederum mit dem Ergebnis, daß keine ventrikuläre Tachykardie vorliegt. Es handelt sich dann um eine n:1 in den Ventrikel übergeleitete atriale Tachykardie. Wird hingegen festgestellt, daß er kein ganzzahliges Vielfaches ist ("Nein" im Schritt S8), besteht eine ventrikuläre Tachykardie.

Der bei annähernder betragsmäßiger Übereinstimmung der RRund PP-Intervalle bei den spontanen Herzaktionen durchzuführende Test mittels einer Folge von Kammer-Stimulationen ("Stim-Test" in Fig. 5b) läuft bei der Anordnung gemäß Fig. 1 bis 4 grundsätzlich wie folgt ab:

Auf die Ausgabe eines die PP-/RR-Übereinstimmung kennzeichnenden Signals CL2 durch die Komparatorstufe 207 hin ändert das Flipflop 31 den Zustand des Ausgangs und damit des Eingangs des (vorher gesperrten) UND-Gatters 37. Bei der nächsten spontanen Kammeraktion trifft zudem über das (ein ankommendes Signal zu diesem Zeitpunkt noch durchschaltende und durch das Signal sperrende) Sperrglied 35 ein Ausgangssignal R von der Kammeraktions-Erfassungseinrichtung 201a ein, woraufhin das UND-Gatter 37 durchschaltet und die Teiler 33, 36, die Zähler 34, 323 sowie die Auswertungseinheit 210b aktiviert.

Hierdurch wird eine Zählung der Zeitsignale t des Zeitgebers 202 durch den Zähler 34 initiiert, die zur Ausgabe eines Stimulations-Steuersignals STIM durch diesen bei Erreichung des über die Berechnungseinheit 32 eingestellten aktuellen Zählwertes führt. Der Zähler-Einstellwert ist in der ersten Stufe des "Stim-Test" das Ergebnis einer Subtraktion eines im Basisdekrementspeicher 322 gespeicherten

Basisdekrementes Δ₀ vom letzten, im Speicher 321 gespeicherten (mittleren) RR-Intervall in der Subtraktionsstufe 325, denn der Zähler 323 steht zu diesem Zeitpunkt auf i = 1, so daß das Basisdekrement in der Multiplikatorstufe 324 nicht vervielfacht wird. Durch Ausgabe des Ausgangssignales - das im übrigen dem 1:n-Teiler 33 zugeführt wird - wird ein Stimulationsimpuls in der Herzkammer zu einem gegenüber der zu erwartenden nächsten spontanen Herzaktion um den Zeitbetrag Δ₁ "verfrühten" Zeitpunkt S₁ ausgegeben. Danach stellt sich der Zähler 34 selbsttätig zurück und erwartet einen neuen Aktivierungsimpuls.

Ein solcher erscheint bei Erfassung einer (m+1)-ten R-Welle, nachdem m R-Wellen durch das Sperrglied 35 unwirksam gemacht worden waren, denn über den 1:m-Teiler 36 wird das Sperrglied nach der m-ten nachfolgenden Kammeraktion (m ist über die Eingabeeinheit 8 programmierbar und wird z.B. auf 8 eingestellt) zurückgesetzt. Daraufhin beginnt der Zähler 34 wieder zu laufen, wobei durch die Berechnungseinheit 32 ein anderer Zählwert eingestellt wird. Dieser ergibt sich entsprechend dem nun erreichten Zählerstand i = 2 des Zählers 323 durch Subtraktion des in der Multiplikatorstufe mit 2 multiplizierten Basisdekrements (Δ₂ = 2Δ₀) vom zuletzt im Speicher 321 gespeicherten RR-Intervall. Der durch das Ausgangssignal des Zählers initiierte Stimulus S₂ hat nun ein um 2Δ₀ verfrühtes Timing. Dieses Vorgehen wird im Rahmen einess Tests für eine programmierte Anzahl n (beispielsweise 5) verschiedener Dekremente wiederholt. Danach gibt der 1:n-Teiler 33 ein Ausgangssignal aus, welches das Flipflop 31 zurücksetzt und damit das Testprogramm beendet.

In jeder Stufe des Tests wird - gesteuert jeweils nach m dazwischenliegenden spontanen Herzaktionen durch ein neues Ausgangsignal des UND-Gatters 37 - der jeweilige Δᵢ-Wert von der Multiplikatorstufe 324 in den Speicher 2084 der Auswertungseinheit 208b und der jeweils korrespondierende Vorhofintervallwert PPᵢ in den Speicher 2082 eingeschrieben. Am Ende des Tests werden - initiiert durch das Ausgangssignal des Teilers 33 - die Speicherinhalte von 2082 und 2084 ausgelesen, und es wird in den Stufen 2086 bis 2088 der Anstieg der Funktionskurve PPᵢ = f(RSᵢ) bestimmt und ermittelt, ob er unterhalb eines im Kriterienspeicher 209b gespeicherten Schwellwertes liegt, d.h. ob die Vorhofintervalle PPi bei ansteigendem RSi im wesentlichen konstant bleiben. Ist dies der Fall, wird durch Ausgabe des Signals CL3 angezeigt, daß eine SVT vorliegt.

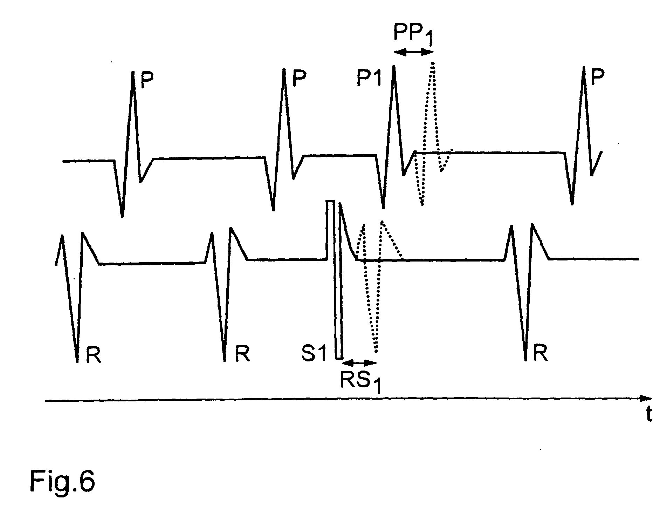

In Fig. 6 ist das Funktionsprinzip des oben beschriebenen Tests anhand der Gegenüberstellung einer Aufzeichnung der Vorhofsignale (oben) und der Kammersignale (unten) mit gemeinsamer Zeitachse illustriert. Es ist zu erkennen, daß ein um den Zeitraum Δ₁ gegenüber einer erwarteten spontanen Kammeraktion R (gestrichelt gezeichnet) verfrühter Kammer-Stimulationsimpuls VES eine gegenüber einer erwarteten spontanen Vorhofaktion P (gestrichelt gezeichnet) um den Betrag PP₁ verfrühte Reaktion P₁ im Vorhof induziert hat.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei anders gearteten Ausführungen Gebrauch macht. Insbesondere sind die angegebenen Blockschaltbilder in keiner Weise beschränkend zu verstehen. So kann der oben skizzierte Test - bei entsprechend geändertem Funktions-Blockschaltbild - auch mit einer Folge fest programmierter Dekrementwerte durchgeführt werden, die sich nicht als ganzzehlige Vielfache eines Basisdekrements ergeben, etwa mit einer Folge 50 ms - 80 ms - 110 ms - 140 ms - 170 ms. Auch der in den Flußdiagrammen gezeigte Ablauf kann variiert werden, indem er vereinfacht wird oder zusätzliche Testkriterien angewandt werden.

## Patentansprüche

1. Vorrichtung zur Erkennung tachykarder Herzrhythmusstörungen, mit einem Stimulationsimpulsgenerator (5), einer über eine Elektrodenleitung mit dem Ausgang des Stimulationsimpulsgenerators verbundenen Kammer-Stimulationselektrode (1a), einer Stimulations-Steuereinrichtung (3), je einer im Vorhof (A) und der Herzkammer (V) eines Herzens (H) anzuordnenden Abfühlelektrode (1b, 1a), einer eingangsseitig mit der Vorhof-Abfühlelektrode (1b) verbundenen Vorhofaktions-Erfassungseinrichtung (201b), einer eingangsseitig mit der Herzkammer-Abfühlelektrode (1a) verbundenen Kammeraktions-Erfassungseinrichtung (201a), einem Zeitgeber (202), einer mit dem Zeitgeber verbundenen Vorhofintervall-Erfassungseinrichtung (203b) zur Erfassung des zeitlichen Abstandes aufeinanderfolgender Vorhofaktionen (P), einer mit dem Zeitgeber verbundenen Kammerintervall-Erfassungseinrichtung (203a) zur Erfassung des zeitlichen Abstandes aufeinanderfolgender Kammeraktionen (R), einer eingangsseitig mit den Ausgängen der Vorhofintervall-Erfassungseinrichtung (203b) und der Kammerintervall-Erfassungseinrichtung (203a) und ausgangsseitig mit dem Eingang der Stimulations-Steuereinrichtung (3) verbundenen Vergleichereinheit (207) zur Ausgabe eines Stimulations-Veranlassungssignals (CL2) im Ergebnis eines Vergleiches der zeitlichen Abstände aufeinanderfolgender Vorhofaktionen und aufeinanderfolgender Kammeraktionen und einer mit dem Ausgang mindestens einer der Vorhofintervall-Erfassungseinrichtung (203b) und der Kammerintervall-Erfassungseinrichtung (203a) verbundenen Auswertungseinrichtung (208b), **dadurch gekennzeichnet, daß** die Stimulations-Steuereinrichtung (3) mit dem Ausgang der Kammeraktions-Erfassungseinrichtung (201a) und dem Zeitgeber (202) verbunden und zur Steuerung der Ausgabe einer Folge von Stimulationsimpulsen (Sᵢ) in solchen Abständen, daß zwischen den Stimulationsimpulsen jeweils mehrere spontane Kammeraktionen auftreten, unter Festlegung eines sich innerhalb der Folge in vorbestimmter Weise ändernden Abstandes (Δᵢ) jedes Kammerstimulationsimpulses zur jeweils vorangehenden oder zur nächsten erwarteten spontanen Kammeraktion ausgebildet ist und die Auswertungseinrichtung (208b) mit der Vorhofintervall-Erfassungseinrichtung (203b) sowie über einen Steuereingang mit der Stimulations-Steuereinrichtung verbunden und zur Erfassung und Auswertung von durch die Kammerstimulationsimpulse (Sᵢ) bewirkten Änderungen der Vorhofintervalle (PPᵢ) und zur Ausgabe eines das Auswertungsergebnis kennzeichnenden Tachykardie-Klassifizierungssignals (CL3) ausgebildet ist.

2. Vorrichtung nach Anspruch 1, **dadurch gekennzeichnet, daß** die Stimulations-Steuereinrichtung (3) eine Stimulationszeitpunkt-Berechnungseinheit (32) umfaßt, die einen Basisdekrementspeicher (322) zur Speicherung eines vorgegebenen Wertes des Kammerstimulations-Zeitabstandes (Δ₀) und Mittel (323 bis 325) zur Berechnung weiterer Werte (Δᵢ) anhand des gespeicherten Wertes oder einen Speicher für eine Mehrzahl vorgegebener Werte des Kammerstimulations-Zeitabstandes aufweist.

3. Vorrichtung nach Anspruch 1 oder 2, **dadurch gekennzeichnet, daß** die Auswertungseinrichtung (208b) einen ersten Speicher (2084) zur Speicherung der Zeitabstände (RSᵢ) einer Mehrzahl vorbestimmter Paare von Kammerstimulationsimpulsen und erwarteten spontanen Kammeraktionen sowie einen zweiten Speicher (2082) zur Speicherung der Zeitabstände einer gleichen Mehrzahl korrespondierender Paare von Vorhofaktionen (PPᵢ) sowie Mittel (2086 bis 2088) zum In-Beziehung-Setzen der Speicherinhalte des ersten und zweiten Speichers (2084, 2082) aufweist.

4. Vorrichtung nach Anspruch 3, **dadurch gekennzeichnet, daß** die Mittel zum In-Beziehung-Setzen eine Korrelatorstufe (2086) und ein mindestens mittelbar mit deren Ausgang verbundenes Differenzierglied (2088) sowie eine Vergleichereinheit (2089) umfassen.

5. Vorrichtung nach Anspruch 4, **dadurch gekennzeichnet, daß** der Vergleichereinheit (2089) ein Kriterienspeicher (209b) zur Speicherung eines Auswertungskriteriums für die Korrelation der Kammerstimulus-Kammeraktions-Zeitabstände (RSᵢ) und der Vorhofaktions-Zeitabstände (PPᵢ) zugeordnet ist.

6. Vorrichtung nach einem der vorhergehenden Ansprüche, **dadurch gekennzeichnet, daß** Mittel (39, 36) zur Einstellung einer vorbestimmten Anzahl (n) von Kammerstimulationsimpulsen (Sᵢ) einer Testfolge und/oder einer vorbestimmten Anzahl (m) von zwischen den Stimulationen einer Testfolge zuzulassenden spontanen Kammeraktionen (R) vorgesehen sind, wobei diese Mittel insbesondere über eine Eingabeeinheit (8) programmierbar sind.

7. Vorrichtung nach einem der vorhergehenden Ansprüche, **dadurch gekennzeichnet, daß** sie als Teil eines implantierbaren Stimulationsgerätes, insbesondere kombinierten Herzschrittmachers/Kardioverters (PMD), ausgebildet ist.

8. Herztherapiegerät mit einer Vorrichtung nach einem der vorhergehenden Ansprüche, **dadurch gekennzeichnet, daß** der Ausgang der Auswertungseinrichtung (208b) mit einem Stimulations- und/oder Defibrillationsimpulsgenerator (5, 6) verbunden ist derart, daß das ausgegebene Tachykardie-Klassifizierungssignal (CL3) zur Steuerung eines Herzschrittmacher- und/oder Defibrillatorbetriebes dient.

## Claims

1. Apparatus for detecting tachycardial cardiac arrhythmia comprising a stimulation pulse generator (5), a chamber stimulation electrode (1a) connected by way of an electrode line to the output of the stimulation pulse generator, a stimulation control device (3), a respective sensing electrode (1a, 1b) to be arranged in the atrium (A) and the ventricle (V) of a heart (H), an atrium action detection device (201b) connected at the input side to the atrium sensing electrode (1b), a ventricle action detection device (201a) connected at the input side to the ventricle sensing electrode (1a), a timer (202), an atrium interval detection device (203) connected to the timer for detecting the time spacing of successive atrium actions (P), a ventricle interval detection device (203a) connected to the timer for detecting the time spacing of successive ventricle actions (R), a comparator unit (207) connected at the input side to the outputs of the atrium interval detection device (203b) and the ventricle interval detection device (203a) and at the output side to the input of the stimulation control device (3) for the output of a stimulation-inducing signal (CL2) as the result of a comparison between the time spacings of successive atrium actions and successive ventricle actions, and an evaluation device (208b) connected to the output of at least one of the atrium interval detection device (203b) and the ventricle interval detection device (203a), **characterised in that** the stimulation control device (3) is connected to the output of the ventricle action detection device (201a) and the timer (202) and is adapted to control the output of a sequence of stimulation pulses (S_{I}) at such spacings that a plurality of spontaneous ventricle actions respectively occur between the stimulation pulses, establishing a spacing (Δ_{I}), which varies within the sequence in a predetermined manner, of each ventricle stimulation pulse with respect to the respective preceding or with respect to the next expected spontaneous ventricle action and the evaluation device (208b) is connected to the atrium interval detection device (203b) and by way of a control input to the stimulation control device and is adapted to detect and evaluate changes in the atrium intervals (PP_{I}), caused by the ventricle stimulation pulses (S_{I}), and to output a tachycardia classification signal (CL3) characterising the evaluation result.

2. Apparatus according to claim 1 **characterised in that** the stimulation control device (3) includes a stimulation time calculation unit (32) which has a base decrement store (322) for storing a predetermined value in respect of the ventricle stimulation time spacing (Δ₀) and means (323 to 325) for calculating further values (Δ_{I}) on the basis of the stored value or a store for a multiplicity of predetermined values in respect of the ventricle stimulation time spacing.

3. Apparatus according to claim 1 or claim 2 **characterised in that** the evaluation device (208b) has a first store (2084) for storing the time spacings (RS_{I}) of a multiplicity of predetermined pairs of ventricle stimulation pulses and expected spontaneous ventricle actions and a second store (2082) for storing the time spacings of an equal multiplicity of corresponding pairs of atrium actions (PP_{I}) and means (2086 to 2088) for relating the store contents of the first and second stores (2084, 2082).

4. Apparatus according to claim 3 **characterised in that** the relating means include a correlator stage (2086) and a differencing member (2088) connected at least indirectly to the output thereof as well as a comparator unit (2089).

5. Apparatus according to claim 4 **characterised in that** associated with the comparator unit (2089) is a criterion store (209b) for the storage of an evaluation criterion for the correlation of the ventricle stimulus-ventricle action time spacings (RS_{I}) and the atrium action time spacings (PP_{I}).

6. Apparatus according to one of the preceding claims **characterised in that** there are provided means (34, 36) for setting a predetermined number (n) of ventricle stimulation pulses (S_{I}) of a test sequence and/or a predetermined number (m) of spontaneous ventricle actions (R) to be permitted between the stimulations of a test sequence, wherein said means are programmable in particular by way of an input unit (8).

7. Apparatus according to one of the preceding claims **characterised in that** it is in the form of part of an implantable stimulation unit, in particular a combined cardiac pacemaker/cardioverter (PMD).

8. A cardiac therapy unit comprising an apparatus according to one of the preceding claims **characterised in that** the output of the evaluation device (208b) is connected to a stimulation and/or defibrillation pulse generator (5, 6) in such a way that the outputted tachycardia classification signal (CLP) serves to control a cardiac pacemaker and/or defibrillator mode of operation.

## Revendications

1. Dispositif d'identification d'arythmies tachycardiques, comportant un générateur (5) d'impulsions de stimulation, une électrode (1a) de stimulation de ventricule, reliée à la sortie du générateur d'impulsions de stimulation par l'intermédiaire d'un conducteur d'électrode, un dispositif de commande de stimulation (3), respectivement une électrode de captage (1b, 1a) devant être disposée dans l'oreillette (A) et dans le ventricule (B) d'un coeur (H), un dispositif (201b) de détection d'action d'oreillette relié côté entrée à l'électrode de captage (1b) de l'oreillette, un dispositif (201a) de détection de l'action du ventricule relié à l'électrode de captage (1a) du ventricule, une minuterie (202), un dispositif (203b) de détection d'intervalles d'oreillette relié à la minuterie pour détecter l'intervalle de temps entre des actions successives (P) de l'oreillette, un dispositif (203a) de détection d'intervalle du ventricule relié à la minuterie pour détecter l'intervalle de temps entre les actions successives (R) du ventricule, une unité formant comparateur (207), qui est relié, côté entrée aux sorties du dispositif (203b) de détection des intervalles de l'oreillette et du dispositif (203a) de détection d'intervalles du ventricule et côté sortie à l'entrée du dispositif de commande de stimulation (3) et sert à délivrer un signal de déclenchement de stimulation (CL2) en tant que résultat d'une comparaison des intervalles de temps des actions successives de l'oreillette et des actions successives du ventricule et un dispositif d'évaluation (208b) relié à la sortie d'au moins l'un du dispositif (203b) de détection d'intervalles de l'oreillette et du dispositif (203b) de détection d'intervalles du ventricule, **caractérisé en ce que** le dispositif de commande de stimulation (3) est relié à la sortie du dispositif (201a) de détection d'action du ventricule et à la minuterie (202) et est conçu pour la commande de la délivrance d'une suite d'impulsions de stimulation (Sᵢ) à des intervalles tels que respectivement plusieurs actions spontanées du ventricule apparaissent entre les impulsions de stimulation, moyennant la détermination d'un intervalle (Δᵢ), qui varie d'une manière prédéterminée à l'intérieur de la suite, de chaque impulsion de stimulation du ventricule par rapport à une action spontanée précédente ou suivante attendue du ventricule et le dispositif d'évaluation (208b) est relié au dispositif (203b) de détection d'intervalles de l'oreillette ainsi que, par l'intermédiaire d'une entrée de commande, au dispositif de commande de stimulation et est agencé de manière à détecter et à évaluer des variations des intervalles d'oreillette (PPᵢ) provoquées par les impulsions (Si) de stimulation du ventricule et pour délivrer un signal de classification de tachycardie (CL3), caractérisant le résultat de l'évaluation.

2. Dispositif selon la revendication 1, **caractérisé en ce que** le dispositif de commande de stimulation (3) comprend une unité (32) de calcul de l'instant de stimulation, et qui comporte une mémoire de décréments de base (322) servant à mémoriser une valeur prédéterminée de l'intervalle de temps (Δ₀) de stimulation du ventricule et des moyens (323 à 325) pour calculer d'autres valeurs (Δᵢ) sur la base de la valeur mesurée ou une mémoire pour une multiplicité de valeurs prédéterminées de l'intervalle de temps de stimulation du ventricule.

3. Dispositif selon la revendication 1 ou 2, **caractérisé en ce que** le dispositif d'évaluation (208b) possède une première mémoire (2084) servant à mémoriser les intervalles de temps (RSᵢ) d'une multiplicité de paires prédéterminées d'impulsions de stimulation du ventricule et des actions spontanées attendues du ventricule, ainsi qu'une seconde mémoire (2082) pour mémoriser les intervalles de temps d'une multiplicité identique de paires correspondantes d'actions d'oreillette (PPᵢ) ainsi que des moyens (2086 à 2088) pour mettre en rapport les contenus des première et seconde mémoires (2084, 2082).

4. Dispositif selon la revendication 3, **caractérisé en ce que** les moyens de mise en rapport comprennent un étage formant corrélateur (2086) et un circuit différentiateur (2088) relié au moins directement à la sortie de l'étage formant corrélateur, ainsi qu'une unité de comparaison (2089).

5. Dispositif selon la revendication 4, **caractérisé par le fait qu'**à l'unité formant comparateur (2089) est associée une mémoire de critère (209b) servant à mémoriser une critère d'évaluation pour la corrélation des intervalles de temps de stimulus du ventricule - action du ventricule (RSᵢ) et des intervalles de temps (PPᵢ) d'action de l'oreillette.

6. Dispositif selon l'une des revendications précédentes, **caractérisé en ce que** des moyens (34, 36) sont prévus pour régler un nombre prédéterminé (n) d'impulsions (Si) de stimulation du ventricule sont une succession de tests et/ou d'un nombre prédéterminé (m) d'actions spontanées (R) du ventricule devant être autorisées entre les stimulations d'une succession de tests, ces moyens étant programmables notamment au moyen d'une unité d'entrée (8).

7. Dispositif selon l'une des revendications précédentes, **caractérisé en ce qu'**elle agencée en tant que partie d'un appareil de stimulation implantable, notamment d'un ensemble combiné stimulateur cardiaque/dispositif de cardioversion (PMD).

8. Appareil de thérapie cardiaque comportant un dispositif selon l'une des revendications précédentes, **caractérisé en ce que** la sortie du dispositif d'évaluation (208b) est reliée à un générateur (5, 6) d'impulsions de stimulation et/ou de défibrillation de telle sorte que le signal délivré de classification de tachycardie (CL3) sert à commander un fonctionnement de stimulateur cardiaque et/ou de défibrillateur.
